# EUROPEAN PATENT SPECIFICATION

(11) **EP 1 557 187 B1**
(45) Date of publication and mention of the grant of the patent: **11.04.2012**
(21) Application number: 03754126.5
(22) Date of filing: 15.10.2003
(51) Int. Cl.: A61M 5/142, A61M 39/28

(54) **CASSETTE FOR INFUSION PUMP**
KASSETTE FÜR INFUSIONSPUMPE
CASSETTE POUR POMPE A PERFUSION

(30) Priority: 24.10.2002 JP 2002310062
(43) Date of publication of application: 27.07.2005
(73) Proprietor: KABUSHIKI KAISHA TOP, Tokyo 120-0035 (JP)
(72) Inventor: OSHIMA, Hiroshi, Adachi-ku, Tokyo 120-0035 (JP)
(74) Representative: Fenlon, Christine Lesley
(86) International application number: PCT/JP2003/013173
(87) International publication number: WO 2004/037322

(56) References cited:
- EP-A1- 0 415 021
- WO-A2-98/13080
- US-A- 4 585 442
- US-A- 5 257 978
- US-A- 5 401 256
- US-A- 5 437 642
- US-A- 5 551 850
- US-A- 5 620 312

## Description

### Technical Field

The present invention relates to a cassette mountable to part of a flexible fluid infusion tube and installable in a fluid infusion pump.

### Background Art

A cassette for a fluid infusion pump of this type is simply mounted to part of a fluid infusion tube and installed in a predetermined position of a fluid infusion pump to allow an infusion by the fluid infusion pump (for example, see Japanese Patent Laid-Open No. 2001-218841, US 5 551 850, WO 98/13080, US 5 257 978 US 5 4 37 642 and US 5 620 312).

In such a fluid infusion pump, because the cassette is detachable from the fluid infusion pump, operations are sometimes performed such that various settings of the fluid infusion pump are made while a temporary infusion by free fall is performed in an emergency, and then the cassette is rapidly installed in the fluid infusion pump to switch to an infusion by the fluid infusion pump.

At this time, operations have to be rapidly performed such that, for example, air bubbles in the fluid infusion tube are manually drawn out in a free flow state, immediately thereafter, the fluid infusion tube is connected to a fluid infusion tube connected to a human body in an anti-free flow state, and then an infusion is started in a free flow state.

In order to address such a situation, the conventional cassette for a fluid infusion pump has a clamp member that presses part of the fluid infusion tube by bias of a coil spring, and also has a key member that locks the clamp member in a position for releasing pressing on the fluid infusion tube. When the key member locks the clamp member, the clamp member releases the pressing on the fluid infusion tube against the bias of the coil spring. When the key member unlocks the clamp member, the clamp member presses the fluid infusion tube by the bias of the coil spring. Thus, the key member locks the clamp member to enter a free flow state, and the key member unlocks the clamp member to enter an anti-free flow state.

However, the key member locks the clamp member against a biasing force of the coil spring, and thus a relatively large biasing force is always applied in a return direction (a clamping direction by the clamp member). This may cause the key member to be detached from a lock position of the clamp member when a vibration or an impact is given to the cassette during, for example, an infusion by free fall in an emergency, to accidentally block the fluid infusion tube.

The clamp member and the key member are components separate from the cassette and movably held by the cassette, thus wear or the like may cause a breakage or a malfunction, and a long period of use may prevent a smooth infusion. Further, components are large in number to require a complicated assembly process duringmanufacturing, thus increasing costs.

In order to eliminate such inconvenience, the invention has an object to provide a cassette for a fluid infusion pump that can reliably switch between a free flow state and an anti-free flow state and easily address an infusion by free fall, and has extremely small number of components and causes few malfunctions.

### Disclosure of the Invention

The invention provides a cassette for a fluid infusion pump mountable to part of a flexible fluid infusion tube and installable in a fluid infusion pump that causes a peristaltic movement in the fluid infusion tube, including: a housing; at least a pair of tube support portions that support the fluid infusion tube in communication with the housing; a tube exposing portion that holds part of the fluid infusion tube in an exposed manner so as to cause the peristaltic movement between the tube support portions; and a clamp portion that releasably clamps the other part of the fluid infusion tube to block a liquid flow in the fluid infusion tube, wherein the clamp portion has a pressing portion provided so as to be diametrically pressed on the fluid infusion tube, a biasing member that biases the pressing portion in a pressing direction on the fluid infusion tube, and a pressing release portion that releases pressing by the pressing portion by a pressing operation against a biasing direction of the biasing member, and the biasing member is provided in the housing movably between a position for biasing the pressing portion, and a position for retracting orthogonally to a biasing direction on the pressing portion to release bias on the pressing portion.

According to the invention, the bias on the pressing portion by the biasing member causes the pressing portion to press the fluid infusion tube to be crashed and blocked, thus reliably preventing an accidental flow of a drug solution in the fluid infusion tube. The simple pressing operation of the pressing release portion allows the release of the pressing on the fluid infusion tube by the pressing portion, thus allowing temporary release of clamping during an operation such as drawing of air bubbles. This allows rapid formation of a free flow state only when necessary such as when air bubbles are manually drawn out of the fluid infusion tube.

Further, movably providing the biasing member allows the formation of the free flow state not only when the pressing release portion is pressed but also when the biasing member is moved to a position for releasing the bias of the pressing portion. Specifically, for example, when an infusion by free fall is performed in an emergency, the biasing member is retracted orthogonally to the biasing direction on the pressing portion to cause the pressing portion to release the pressing on the fluid infusion tube without any biasing force applied from the biasing member. This allows the free flow state to be more reliably kept as compared with the case where the pressing on the fluid infusion tube is released against the biasing force with the biasing force applied as is conventional, and more reliably avoids returning to an anti-free flow state by a vibration or an impact. Besides, for example, moving the biasing member to the position for releasing the bias on the pressing portion when not in use such as when shipped can prevent permanent deformation of the fluid infusion tube in the clamp portion into a crushed state.

In the invention, the pressing portion is provided on a tip of a first arm having a base joined to the housing and swingably extending toward the fluid infusion tube, the pressing release portion is provided on a tip of a second arm having a base joined to the housing and swingably extending toward the fluid infusion tube in parallel with the first arm of the pressing portion, the biasing member is a leaf spring and provided movably along the first arm between a biasing position for abutting on the pressing portion to bias the pressing portion and a biasing release position for releasing abutment on the pressing portion to release pressing on the fluid infusion tube by the pressing portion, and the first arm has a cam portion that slidingly contacts the biasing member when the biasing member moves from the biasing release position to the biasing position to swing the first arm in a direction of pressing the pressing portion on the fluid infusion tube.

The pressing portion is joined to the housing via the first arm, the pressing release portion is joined to the housing via the second arm, and thus the pressing portion and the pressing release portion are operated by swings of the first arm and the second arm. This allows operations of the pressing portion and the pressing release portion in little contact with the housing, thus reducing wear of the pressing portion and the pressing release portion and preventing a malfunction caused by the wear.

Further, simply moving the biasing member from the biasing release position to the biasing position allows a smooth swing of the first arm via the cam portion in a pressing direction on the fluid infusion tube, and allows extremely easy formation of a biasing state of the pressing portion.

Preferably, the housing can be divided into a first side half having the pressing portion orthogonally to a longitudinal direction of the fluid infusion tube supported via the tube support portion, and a second side half having the pressing release portion, and the first side half and the second side half are integrally connected via an engagement portion where the first side half and the second side half engage each other.

Specifically, when the cassette is assembled, the fluid infusion tube is held between the first side half and the second side half, and the first side half and the second side half are connected to easily form the housing. The pressing portion is joined to the first side half, and the pressing release portion is joined to the second side half, thus extremely reducing the number of components, allowing easy assembly, and reducing production costs.

### Brief Description of the Drawings

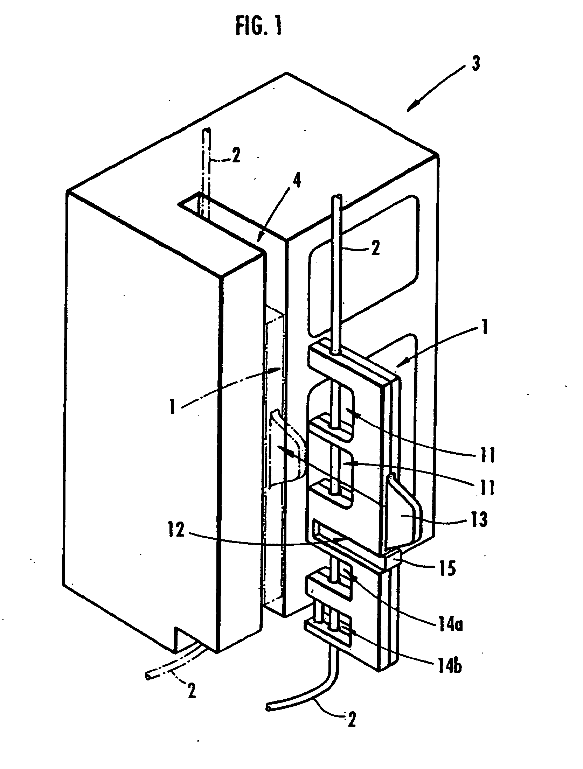

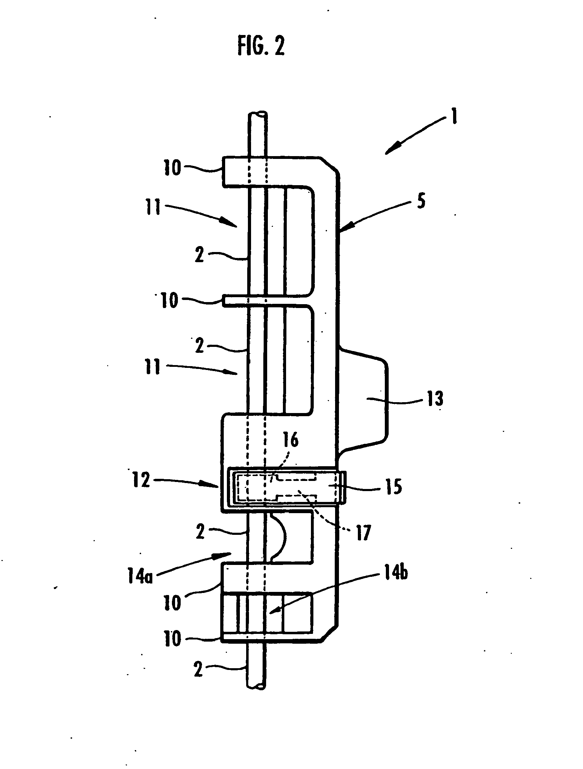

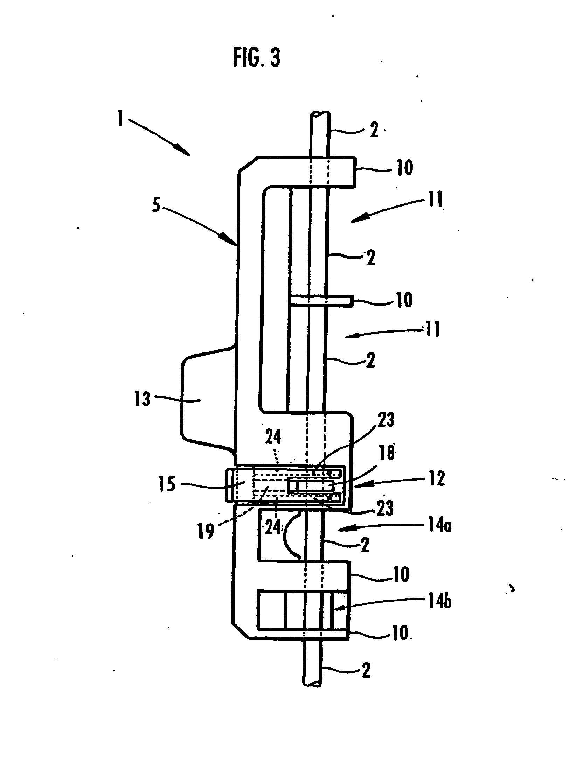

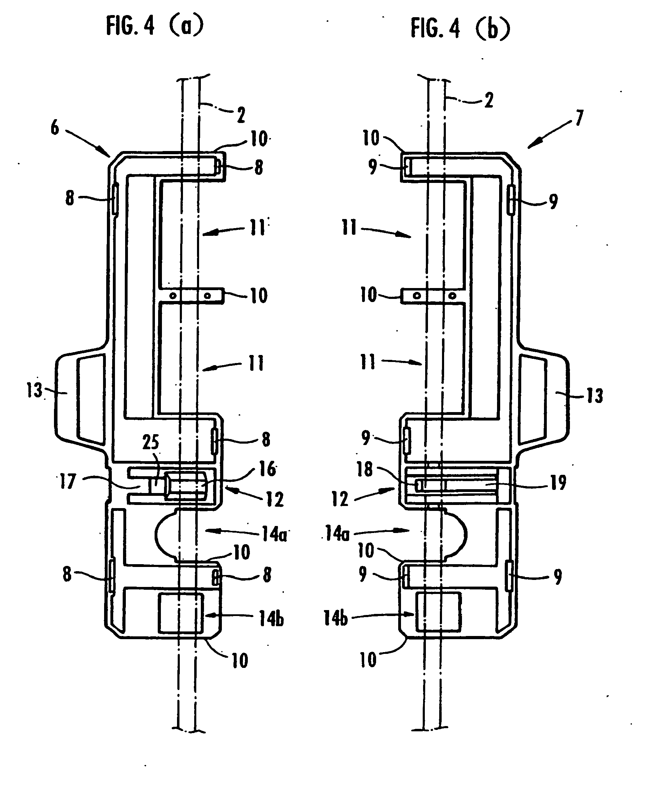

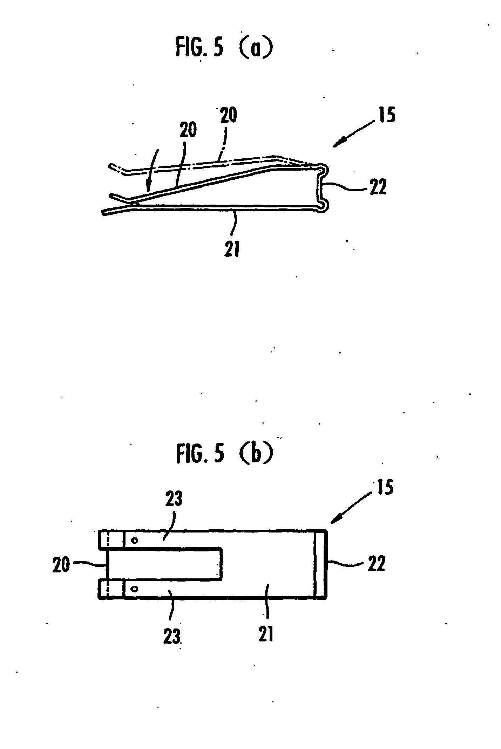

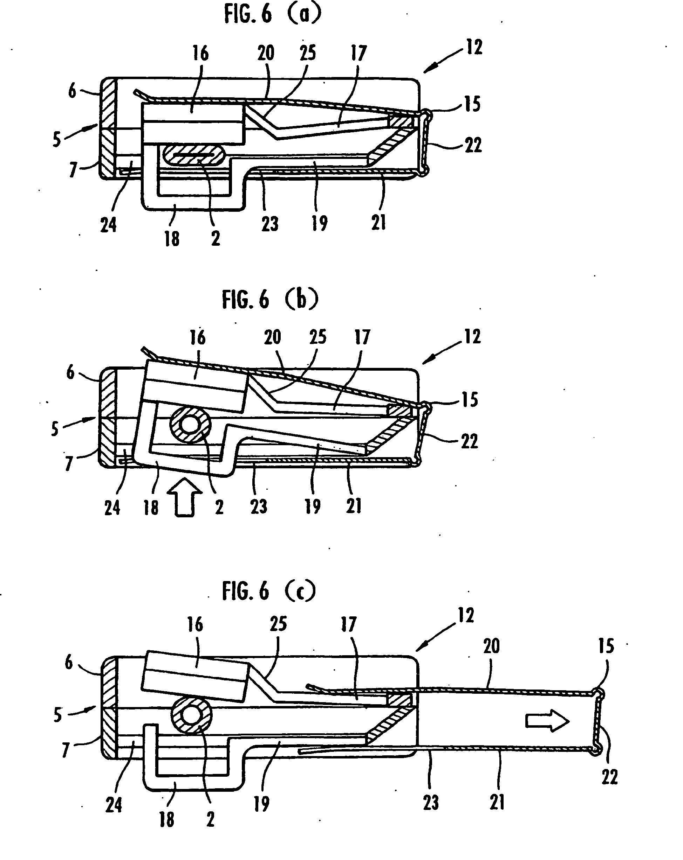

Fig. 1 illustrates a state of use of a cassette according to an embodiment, Fig. 2 is an illustrative side view of one side of the cassette, Fig. 3 is an illustrative side view of the other side of the cassette, Fig. 4(a) illustrates an inner surface side of a first side half of a disassembled cassette, Fig. 4(b) illustrates an inner surface side of a second side half of the disassembled cassette, Fig. 5 illustrates a shape of a biasing member, and Fig. 6 illustrates an operation of a clamp portion.

### Best Mode for Carrying out the Invention

An embodiment of the invention will be described with reference to the drawings.

As shown in Figure 1, a cassette 1 according to the embodiment is mounted to part of a flexible fluid infusion tube 2, and inserted into an insertion slit 4 of the fluid infusion pump 3 and completely installed in the fluid infusion pump 3. The fluid infusion pump 3 has an unshown peristaltic unit including a plurality of fingers that cause a peristaltic movement in the fluid infusion tube 2 supported by the cassette 1.

The cassette 1 has a housing 5 made of synthetic resin as shown in Figures 2 and 3. The housing 5 includes a first side half 6 shown in Figure 4(a) and a second side half 7 shown in Figure 4(b) having a shape corresponding to the first side half 6, and a plurality of locking nails 8 provided on a periphery of the first side half 6 and locking holes 9 provided on a periphery of the second side half 7 are engaged with each other and integrally connected.

The housing 5 has a plurality of tube support portions 10 as shown in Figures 2 and 3. The tube support portions 10 support the fluid infusion tube 2 so as to hold it therebetween when the first side half 6 and the second side half 7 are connected.

The housing 5 also has a first tube exposing portion 11 that exposes part of the fluid infusion tube 2 in a position corresponding to the peristaltic unit of the fluid infusion pump, a clamp portion 12 that clamps the other part of the fluid infusion tube 2, and a grip portion 13 that protrudes from part of the housing 5. Further, a second tube exposing portion 14a and a third tube exposing portion 14b are provided adjacent to the clamp portion 12.

The second tube exposing portion 14a is provided in a position corresponding to an unshown blocking sensor provided in the fluid infusion pump, and at the second tube exposing portion 14a, the blocking sensor can sense a liquid flow stop state in the fluid infusion tube 2 caused by bending of the fluid infusion tube 2 or blocking with a needle. The third tube exposing portion 14b is provided in a position corresponding to an unshown air bubble sensor provided in the fluid infusion pump, and at the third tube exposing portion 14b, the air bubble sensor can sense air bubbles moving in the fluid infusion tube 2.

The clamp portion 12 has, as shown in Figure 2, a below described leaf spring 15 made of metal as a biasing member, and a pressing portion 16 that is pressed on the fluid infusion tube 2 by bias of the spring 15. As shown in Figure 4(a), the pressing portion 16 is provided integrally with the first side half 6 of the housing 5 via a first arm 17. The first arm 17 can swing by elastic bending thereof.

The clamp portion 12 has a pressing release portion 18 facing the pressing portion 16 as shown in Figure 3. As shown in Figure 4(b), the pressing release portion 18 is provided integrally with the second side half 7 of the housing 5 via a second arm 19. The second arm 19 is extended in parallel with the first arm 17, and can swing by elastic bending thereof. As shown in Figures 6(a) to 6(c), the pressing release portion 18 is bent into a Π shape so as to house the fluid infusion tube 2, and a tip thereof abuts against the pressing portion 16.

As shown in Figure 5(a), the spring 15 is bent into a substantial Π shape so that a first extending portion 20 and a second extending portion 21 face each other via a joining portion 22. This causes a biasing force in a direction of the first extending portion 20 moving close to the second extending portion 21. The second extending portion 21 has a pair of extending portions 23 extending in parallel as shown in Figure 5(b). The first extending portion 20 of the spring 15 abuts on the pressing portion 16 to bias the pressing portion 16 as shown in Figures 2 and 6(a). The second extending portion 21 of the spring 15 applies no pressing force to the pressing release portion 18, because the pressing release portion 18 is placed between the pair of extending portions 23. As shown in Figure 3, the extending portions 23 abut against a pair of stepped portions 24 formed along and on both sides of the pressing release portion 18 and the second arm 19 , and receive the bias of the first extending portion 20 of the spring 15 to reliably cause the biasing force of the first extending portion 20. Further, as described below, the spring 15 is movable along the first arm 17 in the clamp portion 12. Specifically, as shown in Figure 6(c), the spring 15 can be slid in a direction orthogonal to a biasing direction on the pressing portion 16. When the spring 15 is slid to a position for biasing the pressing portion 16, the first extending portion 20 of the spring 15 moves along a cam portion 25 provided on the first arm 17, thus allowing smooth formation of a state of biasing the pressing portion 16 as shown in Figure 6(a).

Figure 6(a) shows a clamping state of the fluid infusion tube 2 in the clamp portion 12. At this time, the first extending portion 20 of the spring 15 biases the pressing portion 16, and the pressing portion 16 crushes the fluid infusion tube 2 to block a flow of a drug solution in the fluid infusion tube 2 (an anti-free flow state). This can prevent a rapid flow of the drug solution in the fluid infusion tube 2, for example, when the cassette 1 is detached from the fluid infusion pump 3 as shown in Figure 1. When the cassette 1 is installed in the fluid infusion pump 3, unshown pressing means provided in the fluid infusion pump 3 presses the pressing release portion 18 , thus as shown in Figure 6(b), the pressing on the fluid infusion tube 2 by the pressing portion 16 is released against the bias by the spring 15, and the fluid infusion tube 2 is housed in the Π shaped pressing release portion 18, thus allowing an infusion by the fluid infusion pump 3 (a free flow state).

Further, for example, air bubbles are sometimes manually drawn out before the cassette 1 is installed in the fluid infusion pump 3, and at this time, manually pressing the pressing release portion 18 allows a temporary flow of the drug solution in the fluid infusion tube 2 as shown in Figure 6(b).

Further, when an infusion by free fall is performed without the fluid infusion pump 3 in an emergency, the spring 15 is slid to the position for releasing the bias on the pressing portion 16 as shown in Figure 6(c). This causes the pressing on the fluid infusion tube 2 by the pressing portion 16 to be released, and allows a smooth infusion by free fall. At this time, the bias of the spring 15 is not applied to the pressing portion 16, thus reliably preventing the fluid infusion tube 2 from being accidentally pressed again by a vibration or an impact. The pressing release state shown in Figure 6(c) when the cassette 1 , is shipped or stored for a long period of time can reliably prevent permanent deformation of the fluid infusion tube 2 into a crushed state, and also prevent the fluid infusion tube 2 from being accidentally pressed by the vibration or the impact when the cassette 1 is shipped or transported.

### Industrial Applicability

The cassette according to the invention is simply mounted to part of a fluid infusion tube and installed in a predetermined position of a fluid infusion pump to allow an infusion by the fluid infusion pump, and also easily address an infusion by free fall.

## Claims

1. A cassette (1) for a fluid infusion pump mountable to part of a flexible fluid infusion tube (2) and installable in a fluid infusion pump that causes a peristaltic movement in the fluid infusion tube, comprising:
a housing (5);
at least a pair of tube support portions (10) for supporting the fluid infusion tube in communication with said housing;
a tube exposing portion (11) for holding part of the fluid infusion tube in an exposed manner so as to allow the fluid infusion pump to cause the peristaltic movement between the tube support portions; and
a clamp portion (12) for releasably clamping the other part of the fluid infusion tube to block a liquid flow in the fluid infusion tube,
wherein said clamp portion comprises: a pressing portion (16) provided so as to be diametrically pressed on the fluid infusion tube, a biasing member (15) for biasing said pressing portion in a pressing direction on the fluid infusion tube, and a pressing release portion (18) for releasing pressing by said pressing portion by a pressing operation against the biasing direction of said biasing member, wherein said biasing member is provided in said housing and is movable in a direction which is orthogonal to the biasing direction between a position for biasing said pressing portion, and a retracted position for releasing bias on said pressing portion;
**characterized in that** said pressing portion is provided on a tip of a first arm (17) having a base joined to the housing and swingably extending toward said fluid infusion tube;
wherein said pressing release portion is provided on a tip of a second arm (19) having a base joined to the housing and swingably extending toward said fluid infusion tube in parallel with the first arm of said pressing portion;
wherein said biasing member comprises a leaf spring (15) and provided movably along said first arm between a biasing position for abutting on said pressing portion to bias said pressing portion and a biasing release position for releasing abutment on said pressing portion to release pressing on the fluid infusion tube by said pressing portion; and
wherein said first arm comprises a cam portion (25) slidingly contacting said biasing member when the biasing member moves from the biasing release position to the biasing position to swing said first arm in a direction of pressing said pressing portion on the fluid infusion tube.

2. The cassette for a fluid infusion pump according to claim 1, wherein said housing can be divided into a first side half (6) having said pressing portion orthogonally to a longitudinal direction of the fluid infusion tube supported via said tube support portion, and a second side half (7) having said pressing release portion, and the first side half and the second side half are connected via an engagement portion where the first side half and the second side half engage each other.

## Patentansprüche

1. Kassette (1) für eine Fluidinfusionspumpe, befestigbar an einem Teil einer elastischen Fluidinfusionsröhre (2) und einbaubar in einer Fluidinfusionspumpe, die eine peristaltische Bewegung in der Fluidinfusionsröhre bewirkt, umfassend
ein Gehäuse (5);
mindestens ein Paar Röhrenstützabschnitte (10) zum Stützen der Fluidinfusionsröhre in Verbindung mit dem Gehäuse;
einen Röhrenfreilegeabschnitt (11) zum Halten eines Teils der Fluidinfusionsröhre in einer freigelegten Position, so dass der Fluidinfusionspumpe ermöglicht wird, die peristaltische Bewegung zwischen den Röhrenstützabschnitten zu bewirken; und
einen Klemmenabschnitt (12) zum lösbaren Festklemmen des anderen Abschnitts der Fluidinfusionsröhre, um einen Flüssigkeitsfluss in der Fluidinfusionsröhre zu blockieren,
wobei der Klemmenabschnitt umfasst einen Druckabschnitt (16), so bereitgestellt, dass er diametral an die Fluidinfusionsröhre angepresst wird, einen Ausrichtebauteil (15) zum Ausrichten des Druckabschnitts in eine Druckrichtung auf der Fluidinfusionsröhre, und einen Drucklöseabschnitt (18) zum Lockern des Drucks durch den Druckabschnitt durch eine Druckausübung gegen die Ausrichtungsrichtung des Ausrichtebauteils, wobei der Ausrichtebauteil im Gehäuse bereitgestellt wird und in eine Richtung beweglich ist, die senkrecht zur Ausrichtungsrichtung zwischen einer Position zum Ausrichten des Druckabschnitts und einer zurückgezogenen Position zum Lösen der Ausrichtung auf den Druckabschnitt;
**dadurch gekennzeichnet, dass** der Druckabschnitt bereitgestellt wird auf einer Spitze eines ersten Arms (17) mit einer mit dem Gehäuse verbundenen Basis und der sich drehbar zur Fluidinfusionsröhre hin erstreckt;
wobei der Drucklöseabschnitt bereitgestellt wird auf einer Spitze eines zweiten Arms (19) mit einer mit dem Gehäuse verbundenen Basis und der sich parallel zum ersten Arm des Druckabschnitts drehbar zur Fluidinfusionsröhre hin erstreckt;
wobei der Ausrichtebauteil eine Blattfeder (15) umfasst und beweglich bereitgestellt ist entlang dem ersten Arm zwischen einer Ausrichtungsposition zum Gegendrücken an den Druckabschnitt zum Ausrichten des Druckabschnitts und einer Ausrichtungslöseposition zum Lockern des Gegendrucks gegen den Druckabschnitt zum Lockern des Drucks auf die Fluidinfusionsröhre durch den Druckabschnitt; und
wobei der erste Arm einen Nockenabschnitt (25) umfasst, der den Ausrichtebauteil gleitbar berührt, wenn sich der Ausrichtebauteil aus der Ausrichtungslöseposition in die Ausrichtungsposition bewegt, um den ersten Arm in eine Richtung des Drückens des Druckabschnitts auf die Fluidinfusionsröhre dreht.

2. Kassette für eine Fluidinfusionspumpe gemäß Anspruch 1, wobei das Gehäuse aufgeteilt werden kann in eine erste Seitenhälfte (6) mit dem Druckabschnitt senkrecht zu einer Längsrichtung der durch den Röhrenstützabschnitt gestützten Fluidinfusionsröhre, und eine zweite Seitenhälfte (7) mit dem Drucklöseabschnitt, wobei die erste Seitenhälfte und die zweite Seitenhälfte über einen Eingriffabschnitt verbunden sind, wobei die erste Seitenhälfte und die zweite Seitenhälfte ineinander eingreifen.

## Revendications

1. Cassette (1) pour une pompe à perfusion de fluide pouvant être montée sur une partie d'un tube de perfusion de fluide souple (2) et pouvant être installée dans une pompe à perfusion de fluide qui provoque un déplacement péristaltique dans le tube de perfusion de fluide, comprenant :
un boîtier (5) ;
au moins une paire de parties de support de tube (10) pour supporter le tube à perfusion de fluide en communication avec ledit boîtier ;
une partie d'exposition de tube (11) pour maintenir une partie du tube de perfusion de fluide d'une manière exposée afin de permettre à la pompe à perfusion de fluide de provoquer le déplacement péristaltique entre les parties de support de tube ; et
une partie pince (12) pour pincer de manière amovible l'autre partie du tube à perfusion de fluide afin de bloquer un écoulement de liquide dans le tube à infusion de fluide,
dans laquelle ladite partie pince comprend : une partie de pression (16) placée de façon à être pressée diamétralement sur le tube à perfusion de fluide, un élément de sollicitation (15) pour pousser ladite partie de pression dans une direction d'enfoncement sur le tube à infusion de fluide, et une partie de libération de pression (18) pour libérer la pression exercée par ladite partie de pression par une opération d'enfoncement contre la direction de sollicitation dudit élément de sollicitation, dans laquelle ledit élément de sollicitation est placé dans ledit boîtier et est mobile dans une direction qui est orthogonale à la direction de sollicitation entre une position servant à pousser ladite partie de pression, et une position rétractée servant à libérer la sollicitation exercée sur ladite partie de pression ;
**caractérisée en ce que** ladite partie de pression est formée sur un bout d'au premier bras (17) ayant une base liée au boîtier et s'étendant de façon pivotante vers ledit tube à perfusion de fluide ;
dans laquelle ladite partie de libération de pression est formée sur un bout d'un deuxième bras (19) ayant une base liée au boîtier et s'étendant de façon pivotante vers ledit tube à perfusion de fluide parallèlement au premier bras de ladite partie de pression ;
dans laquelle ledit élément de sollicitation comprend un ressort à lame (15) et est placé de façon mobile le long dudit premier bras entre une position de sollicitation où il doit prendre appui sur ladite partie de pression pour enfoncer ladite partie de pression et une position de libération de sollicitation pour dégager l'appui sur ladite partie de pression pour libérer la pression exercée sur le tube à perfusion de fluide par ladite partie de pression ; et
dans laquelle ledit premier bras comprend une partie came (25) qui est en contact glissant avec ledit élément de sollicitation quand l'élément de sollicitation se déplace de la position de libération de sollicitation à la position de sollicitation pour faire pivoter ledit premier bras dans une direction d'enfoncement de ladite partie de pression sur le tube à perfusion de fluide.

2. Cassette pour pompe à perfusion de fluide selon la revendication 1, dans laquelle ledit boîtier peut être divisé en une première moitié latérale (6) comportant ladite partie de pression orthogonalement à une direction longitudinale du tube à perfusion de fluide supporté par l'intermédiaire de ladite partie de support de tube, et une deuxième moitié latérale (7) comportant ladite partie de libération de pression, et la première moitié latérale et la deuxième moitié latérale sont reliées par l'intermédiaire d'une partie d'accrochage où la première moitié latérale et la deuxième moitié latérale s'accrochent entre elles.
